Europäisches Patentamt

European Patent Office

Office européen des brevets

(11) Numéro de publication: **0 428 452 A1**

## (12) DEMANDE DE BREVET EUROPEEN

(21) Numéro de dépôt: 90403240.6

(22) Date de dépôt: 16.11.90

(51) Int. Cl.⁵: **A61B 17/16**

(30) Priorité: 16.11.89 FR 8915049

(43) Date de publication de la demande:
22.05.91 Bulletin 91/21

(84) Etats contractants désignés:
AT BE CH DE DK ES FR GB GR IT LI LU NL SE

(71) Demandeur: **CENDIS MEDICAL SARL**
**6, Avenue de la Porte de Montrouge**
**F-75014 Paris(FR)**

(72) Inventeur: **Choukroun, René**
**6, place de Clichy**
**F-75009 Paris(FR)**

(74) Mandataire: **Hasenrader, Hubert et al**
**Cabinet BEAU DE LOMENIE 55, rue**
**d'Amsterdam**
**F-75008 Paris(FR)**

(54) Dispositif de visée pour ligamentoplastie et son procédé de fabrication.

(57) Dispositif de visée pour ligamentoplastie comprenant une tige rigide amovible (1, 1´, 1´´) montée sur un rail de support (2) en forme de portion de cercle constitué de deux branches séparables (2a, 2b) articulées autour d'une charnière à axe radial et solidaire d'un organe de visée radiale (5, 5´) de telle sorte que l'extrémité libre de la tige rigide vienne se placer dans l'alignement de l'organe de visée, caractérisé en ce que ladite tige rigide amobible est déplaçable sur ledit rail (2) et en ce que ladite charnière (9) à axe radial comprend un tube fileté (10) à base crantée réalisé perpendiculairement sur l'une des branches au niveau de l'articulation et destiné à venir s'emboîter dans un canon creux (11) à base crantée réalisé perpendiculairement sur l'autre branche et un organe de serrage (12) dudit tube fileté (10) dans ledit canon creux (11).

Fig-1

EP 0 428 452 A1

# DISPOSITIF DE VISÉE POUR LIGAMENTOPLASTIE ET SON PROCÉDÉ DE FABRICATION

La présente invention concerne un dispositif de visée pour la réalisation d'une ligamentoplastie du genou ou de toute autre articulation ainsi que le procédé de fabrication d'un tel dispositif.

On sait que le remplacement de ligaments rompus ou endommagés nécessite la fixation osseuse du ligament de remplacement ou de renfort (ligament artificiel ou naturel) qui peut être de nature permanente ou de nature biodégradable. Cette fixation peut impliquer que l'on réalise un canal intra osseux.

Il existe déjà des viseurs qui comportent une tige rigide rectiligne terminée à une extrémité par un crochet et dont l'autre extrémité est montée radialement et de façon déplaçable sur un rail de support en forme de portion de cercle solidaire d'un organe de visée radiale.

Cependant, de tels viseurs n'offrent pas toujours la maniabilité requise dans toutes les positions articulaires et ne permettent pas la visée dans toutes les orientations de l'espace.

La présente invention a pour but de résoudre ce problème technique, grâce à un dispositif de visée destiné principalement aux techniques chirurgicales de ligamentoplasties articulaires par voie arthrotomique ou arthroscopique.

Ce but est atteint conformément à l'invention par un dispositif de visée pour ligamentoplastie comprenant une tige rigide amovible montée sur un rail de support en forme de portion de cercle constitué de deux branches séparables articulées autour d'une charnière à axe radial et solidaire d'un organe de visée radiale de telle sorte que l'extrémité libre de la tige rigide vienne se placer dans l'alignement de l'organe de visée, caractérisé en ce que ladite tige rigide amovible est déplaçable sur ledit rail et en ce que ladite charnière à axe radial comprend un tube fileté à base crantée réalisé perpendiculairement sur l'une des branches au niveau de l'articulation et destiné à venir s'emboîter dans un canon creux à base crantée réalisé perpendiculairement sur l'autre branche et un organe de serrage dudit tube fileté dans ledit canon creux.

Selon encore une autre caractéristique, ledit rail de support comporte au moins une rainure interne servant au déplacement de ladite tige rigide.

Selon un autre aspect de l'invention, ledit organe de visée comprend un premier tube présentant une extrémité avant effilée et une extrémité arrière munie d'une butée, ledit premier tube étant adapté pour s'emboîter de façon amovible et coulissante dans un second tube plus court et dont l'extrémité arrière est munie d'une contre-butée destinée à coopérer avec la butée dudit premier tube pour maintenir ledit premier tube dans ledit second tube.

Selon encore un autre aspect, ledit rail de support est rendu solidaire de l'organe de visée au moyen d'un collier cylindrique orienté radialement et destiné à recevoir de façon amovible lesdits premier et second tubes de l'organe de visée et au moyen d'un élément de blocage dudit organe de visée dans ledit collier.

Selon une autre caractéristique de réalisation ci-dessus, ladite tige rigide est courbe et est montée au moyen d'un curseur sur ladite branche comportant ledit canon creux et ladite tige rigide a son extrémité libre qui se termine par un oeilleton comportant un orifice destiné à venir se placer dans l'alignement de l'organe de visée.

Un autre objet de l'invention est une branche constituant un moyen essentiel du dispositif précédent, caractérisée en ce qu'elle est en forme de portion de cercle et comporte un canon creux à base crantée s'étendant perpendiculairement vers l'extérieur de la portion de cercle au niveau de l'une de ses extrémités.

Encore un autre objet de l'invention est un procédé de fabrication d'un dispositif de visée pour ligamentoplastie comprenant une tige rigide amovible montée sur un rail de support en forme de portion de cercle solidaire d'un organe de visée radiale de telle sorte que l'extrémité libre de la tige rigide vienne se placer dans l'alignement de l'organe de visée, ledit rail en forme de portion de cercle étant constitué de deux branches séparables articulées autour d'une charnière à axe radial comprenant un tube fileté à base crantée réalisé perpendiculairement sur l'une des branches au niveau de l'articulation et destiné à venir s'emboîter dans un canon creux à base crantée réalisé perpendiculairement sur l'autre branche et un organe de serrage dudit tube fileté dans ledit canon creux, caractérisé en ce qu'on sépare ladite branche comportant le canon creux de l'autre branche et on emboîte l'organe de visée dans ledit canon creux.

Dans les ligamentoplasties du genou, le dispositif de visée de l'invention peut donc être utilisé à la fois pour la ligamentoplastie du croisé antérieur, et pour la ligamentoplastie du croisé postérieur.

L'invention sera mieux comprise à la lecture de la description qui va suivre accompagnée des dessins annexés, sur lesquels :

- la figure 1 représente une vue générale du dispositif de l'invention appliqué à un genou ;
- la figure 2 représente un mode de réalisation du dispositif de l'invention ;
- la figures 3 et 4 représentent une variante de réalisation du dispositif de l'invention.

Le dispositif de visée de l'invention est un instrument chirurgical permettant de contrôler et de réaliser avec précision le forage d'un tunnel osseux dans une direction déterminée pour y introduire ensuite un ligament artificiel ou la greffe d'un ligament naturel, entre le tibia (T) et le fémur (F).

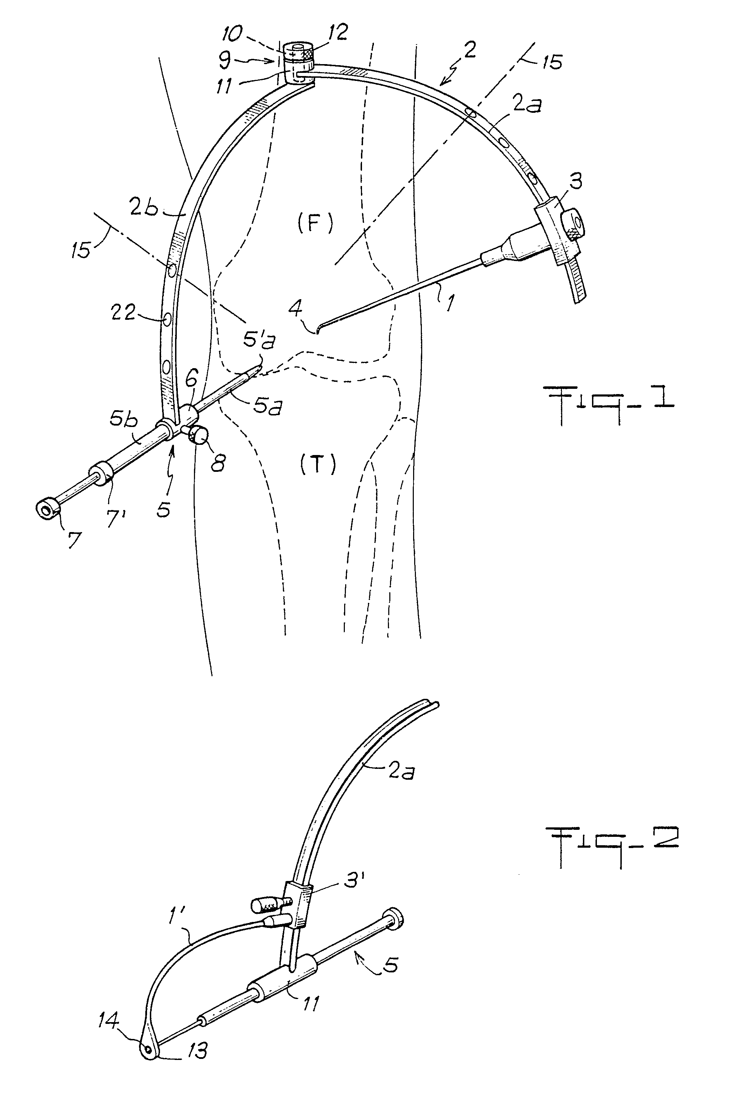

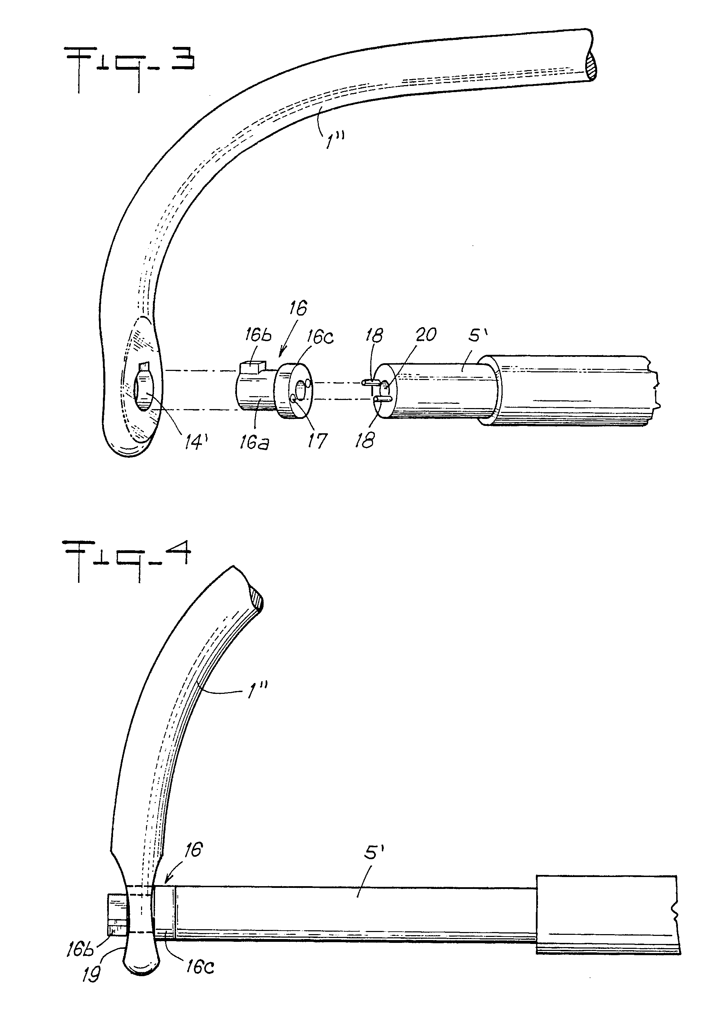

Ce dispositif peut être utilisé soit pour la ligamentoplastie du ligament croisé antérieur, selon le mode de réalisation de la figure 1, soit pour la ligamentoplastie du ligament croisé postérieur, selon le mode de réalisation des figures 2, 3 et 4.

Le dispositif tel que représenté sur la figure 1 comprend une tige rigide amovible 1, montée de façon déplaçable sur un rail de support 2 en forme de portion de cercle. La tige rigide 1 est rectiligne et montée radialement au moyen d'un curseur 3 sur le rail de support 2 en se terminant à son extrémité libre par un crochet 4 (qui est sur la figure 1 perpendiculaire à la tige 1). On peut concevoir également un autre mode de réalisation dans lequel la tige rigide possède une extrémité effilée sans crochet.

Le rail de support 2 comporte au moins une rainure interne de guidage servant au déplacement du curseur 3 supportant la tige 1.

Le rail de support 2 en forme de portion de cercle est solidaire d'un organe de visée radiale 5. Le rail de support 2 est rendu solidaire de l'organe de visée 5 au moyen d'un collier cylindrique 6 orienté radialement et situé à une des extrémités du rail de support 2.

L'organe de visée 5 comprend un premier tube 5a présentant une extrémité avant effilée et une extrémité arrière munie d'une butée 7. Le premier tube 5a est adapté pour s'emboîter de façon amovible et coulissante dans un second tube 5b plus court que le premier tube 5a et dont l'extrémité arrière est munie d'une contre-butée 7' destinée à coopérer avec la butée 7 du premier tube 5a pour maintenir le premier tube 5a dans le second tube 5b.

Le collier cylindrique 6 reçoit de façon amovible le premier tube 5a et le second tube 5b de l'organe de visée 5 et est associé à un élément de blocage 8 servant à fixer l'organe de visée 5 dans le collier 6. Cet élément de blocage 8 pourra par exemple être constitué d'une vis molletée ou de tout autre moyen analogue.

Le rail de support 2 est constitué de deux branches séparables 2a, 2b articulées autour d'une charnière 9 à axe radial, de telle sorte que les deux branches 2a, 2b peuvent pivoter l'une par rapport à l'autre autour dudit axe radial avec un angle compris entre 0 et 180°.

La tige 1 peut donc prendre toute position radiale à partir d'un point quelconque de l'espace situé sur une portion de sphère.

La tige 1 s'étend radialement dans le plan défini par la portion de cercle du rail 2. Cette position de la tige 1 est assurée au moyen du curseur 3 qui coulisse sur la branche 2a mais qui ne doit pas tourner autour de cette branche. Ce résultat est obtenu en réalisant, par exemple, la branche 2a avec une section rectangulaire coopérant avec une section correspondante du curseur 3.

La charnière 9 comprend un tube fileté 10 à base crantée réalisé perpendiculairement sur l'une des branches 2a, 2b au niveau de l'articulation et destiné à venir s'emboîter dans un canon creux 11 à base crantée réalisé perpendiculairement sur l'autre branche. L'organe de serrage 12 tel qu'un écrou molleté se vissant sur le col du tube fileté 10 permet de maintenir le tube fileté 10 dans le canon creux 11 après pivotement pour conserver le positionnement correct de la branche 2a par rapport à la branche 2b.

Selon un mode de réalisation particulièrement avantageux, la charnière 9 s'étend vers l'extérieur du rail 2 et est située entre les deux branches 2a, 2b, sensiblement au milieu du rail de support 2 et l'organe de visée 5 est monté sensiblement à l'extrémité de l'une des deux branches 2a, 2b articulées, et de préférence à l'extrémité de la branche 2b comportant le tube fileté 10.

Selon un mode de réalisation avantageux, les branches 2a, 2b comportent des perforations 22 destinées à l'introduction de broches 15 permettant la stabilisation et le maintien du dispositif en place, une fois qu'un positionnement correct a été obtenu.

L'utilisation du viseur selon l'invention s'effectue de la manière suivante : on introduit dans ledit genou la tige 1 et l'on plante le crochet terminal 4, par exemple, au centre de la zone du plateau tibial où s'attache une extrémité du ligament ; le viseur étant ainsi mis en place, on déplace progressivement le curseur 3 de façon que la direction de l'organe de visée soit optimale pour réaliser le perçage de l'os ; on fixe alors le curseur 3 dans sa position optimale sur le rail 2 ; on stabilise le dispositif au moyen des broches 15, on adapte ensuite, si cela n'a pas déjà été fait, les tubes convenables dans ledit organe de visée et on perce, selon l'axe ainsi déterminé, l'os jusqu'à ce que l'outil de perçage ressorte, sur le plateau tibial, au niveau de l'extrémité de la tige 1 matérialisée par le crochet 4.

Le dispositif de visée représenté sur la figure 2 est plus particulièrement utilisé pour la ligamentoplastie du croisé postérieur.

Nous avons déjà vu que la branche 2a est elle-même en forme de portion de cercle et comporte un canon creux 11 à base crantée s'étendant perpendiculairement vers l'extérieur de la portion de cercle au niveau de l'une de ses extrémités.

Dans le mode de réalisation de la figure 2, la branche 2a comportant le canon creux 11 est séparée de la branche 2b comportant le tube fileté 10.

La branche 2a est rendue solidaire de l'organe de visée en emboîtant ce dernier dans le canon creux 11. Ce mode de réalisation suppose que le diamètre intérieur du canon creux soit légèrement supérieur au diamètre extérieur du second tube 5b de l'organe de visée.

La tige rigide 1' est réalisée de façon courbe et est montée au moyen d'un curseur 3 sur la branche 2a comportant un canon creux 11. La tige rigide 1' a son extrémité libre qui se termine par un oeilleton ou méplat 13 comportant un orifice 14 destiné à venir se placer dans l'alignement de l'organe de visée 5.

Dans le mode de réalisation de la figure 2, le dispositif de visée est mis en oeuvre à l'extérieur de la portion de cercle constituant la branche 2a, à l'inverse du dispositif de la figure 1, qui est mis en oeuvre avec le genou à l'intérieur de la portion de cercle constituant le rail de support 2.

Dans ce mode de réalisation, la tige rigide courbe 1' et l'organe de visée 5 s'étendent donc à partir du curseur 3 vers l'extérieur de la portion de cercle, ce qui rend le dispositif plus adapté et plus maniable, compte tenu de la position du genou sur lequel on opère.

Selon une variante avantageuse de réalisation telle qu'illustrée par la figure 3, la tige rigide courbe 1" comporte à son extrémité libre un orifice 14' conformé en "serrure", c'est-à-dire avec une découpe asymétrique. L'orifice 14' se trouve dans l'alignement de l'organe de visée 5' dont l'extrémité est munie d'une clé 16 amovible dont le profil correspond aux contours de l'orifice 14'.

La clé 16 est constituée d'un corps cylindrique 16a dont la longueur est supérieure à l'épaisseur de la tige rigide 1" au niveau de l'orifice 14' et qui est pourvu, à son extrémité avant, d'un ergot 16b et, à son extrémité arrière, d'un épaulement cylindrique 16c dont le diamètre est supérieur à celui du corps 16a. L'ergot 16b et l'épaulement 16c s'étendent à partir de la paroi latérale externe du corps 16a.

De cette façon, la clé 16 supportée par l'extrémité de l'organe de visée 5 est introduite par l'opérateur dans l'orifice 14' puis tournée d'un angle compris entre 90° et 180° afin de la bloquer dans ledit orifice comme représenté sur la figure 4. La clé 16 est retenue dans cette position grâce à l'ergot 16b et à l'épaulement 16c qui sont donc disposés de part et d'autre de l'extrémité de la tige 1", l'ergot 16b venant en appui de calage contre une surépaisseur 19 de l'extrémité de la tige 1".

L'épaulement cylindrique 16c de la clé 16 comporte, sur le bord plein de sa face externe, des orifices 17 destinés à l'introduction de dents 18 s'étendant à partir de l'extrémité avant de l'organe de visée 5' en formant ainsi un embout mâle. Les dents 18 coopèrent avec les orifices 17 pour permettre le guidage, l'introduction et le verrouillage de la clé 16 dans l'orifice 14'.

Une fois que la clé 16 est verrouillée en position dans l'orifice 14', l'organe de visée 5' peut être translaté vers l'arrière et retiré du dispositif.

Ce mode de réalisation peut être utilisé de façon avantageuse en utilisant la clé comme moyen passe-fil pour la pose dudit fil dans un canal osseux.

A cet effet, le fil est tout d'abord introduit au travers de l'organe de visée 5' qui comporte à cet effet un conduit interne 20 puis attaché au niveau de la clé 16 se trouvant à l'extrémité de l'organe 5'. La clé 16 est ensuite verrouillée dans l'orifice 14' puis séparée de l'organe de visée 5' en restant attaché au fil qui se trouve ainsi en place dans le canal osseux.

Le dispositif de l'invention peut être réalisé en Inox ou en titane ou bien encore avec un alliage chrome-cobalt.

## Revendications

1. Dispositif de visée pour ligamentoplastie comprenant une tige rigide amovible (1, 1', 1") montée sur un rail de support (2) en forme de portion de cercle constitué de deux branches séparables (2a, 2b) articulées autour d'une charnière à axe radial et solidaire d'un organe de visée radiale (5, 5') de telle sorte que l'extrémité libre de la tige rigide vienne se placer dans l'alignement de l'organe de visée, caractérisé en ce que ladite tige rigide amobibile est déplaçable sur ledit rail (2) et en ce que ladite charnière (9) à axe radial comprend un tube fileté (10) à base crantée réalisé perpendiculairement sur l'une des branches au niveau de l'articulation et destiné à venir s'emboîter dans un canon creux (11) à base crantée réalisé perpendiculairement sur l'autre branche et un organe de serrage (12) dudit tube fileté (10) dans ledit canon creux (11).

2. Dispositif selon la revendication 1, caractérisé en ce que la charnière (9) s'étend vers l'extérieur du rail de support (2) et est en particulier située sensiblement au milieu du rail de support (2) en portion de cercle.

3. Dispositif selon l'une des revendications 1 ou 2, caractérisé en ce que l'organe de visée (5) radiale est monté sensiblement à l'extrémité de l'une des deux branches articulées (2a, 2b).

4. Dispositif selon l'une des revendications 1 à 3, caractérisé en ce que ledit rail de support (2) comporte au moins une rainure interne servant au déplacement de ladite tige rigide (1, 1').

5. Dispositif selon l'une des revendications 1 à 4, caractérisé en ce que ledit organe de visée (5) comprend un premier tube (5a) présentant une extrémité avant effilée et une extrémité arrière munie d'une butée (7), ledit premier tube (5a) étant adapté pour s'emboîter de façon amovible et coulissante dans un second tube (5b) plus court et dont l'extrémité arrière est munie d'une contrebutée (7') destinée à coopérer avec la butée (7) dudit premier tube (5a) pour maintenir ledit premier tube dans ledit second tube (5b).

6. Dispositif selon l'une des revendications 3 ou 5, caractérisé en ce que ledit rail de support est rendu solidaire de l'organe de visée au moyen d'un collier cylindrique (6) orienté radialement et destiné à recevoir de façon amovible lesdits premier et second tubes de l'organe de visée (5) et au moyen d'un élément de blocage (8) dudit organe de visée dans ledit collier.

7. Dispositif selon l'une des revendications précédentes, caractérisé en ce que ladite tige rigide (1) est rectiligne et montée radialement au moyen d'un curseur (3) sur ledit rail de support (2) en se terminant à son extrémité libre par un crochet (4).

8. Dispositif selon l'une quelconque des revendications précédentes, caractérisé en ce que ladite branche (2b) comportant le tube fileté (10) à base crantée est la branche solidaire de l'organe de visée (5).

9. Dispositif selon l'une des revendications précédentes, caractérisé en ce que les branches (2a, 2b) comportent des perforations (22) destinées à l'introduction de broches de maintien, caractérisé en ce qu'on sépare ladite branche (2a) comportant le canon creux (11) de l'autre branche (2b) et on emboîte l'organe de visée (5) dans ledit canon creux (11).

10. Dispositif selon l'une des revendications 1 à 6, caractérisé en ce que ladite tige rigide (1', 1'') est courbe et est montée au moyen d'un curseur (3') sur ladite branche comportant ledit canon creux.

11. Dispositif selon la revendication 10, caractérisé en ce que ladite tige rigide (1', 1'') a son extrémité libre qui se termine par un oeilleton (13) comportant un orifice (14, 14') destiné à venir se placer dans l'alignement de l'organe de visée (5, 5').

12. Dispositif selon la revendication 10, caractérisé en ce que la tige rigide (1'') comporte à son extrémité libre un orifice (14') asymétrique, dans lequel une clé (16) formant moyen passe-fil est destinée à être verrouillée.

13. Dispositif selon la revendication 12, caractérisé en ce que ladite clé (16) est montée de manière amovible à l'extrémité de l'organe de visée (5').

14. Branche constituant un moyen essentiel du dispositif selon l'une quelconque des revendications 1 à 13, caractérisée en ce qu'elle est en forme de portion de cercle et comporte un canon creux (11) à base crantée s'étendant perpendiculairement vers l'extérieur de la portion de cercle au niveau de l'une de ses extrémités et destiné à recevoir un organe de visée (5, 5').

15. Procédé de fabrication d'un dispositif de visée pour ligamentoplastie comprenant une tige rigide amovible (1, 1', 1'') montée sur un rail de support (2) en forme de portion de cercle solidaire d'un organe de visée radiale (5, 5') de telle sorte que l'extrémité libre de la tige rigide vienne se placer dans l'alignement de l'organe de visée, ledit rail en forme de portion de cercle étant constitué de deux branches séparables (2a, 2b) articulées autour d'une charnière à axe radial comprenant un tube fileté (10) à base crantée réalisé perpendiculairement sur l'une des branches au niveau de l'articulation et destiné à venir s'emboîter dans un canon creux (11) à base crantée réalisé perpendiculairement sur l'autre branche et un organe de serrage (12) dudit tube fileté (10) dans ledit canon creux (11), caractérisé en ce qu'on sépare ladite branche (2a) comportant le canon creux (11) de l'autre branche (2b) et on emboîte l'organe de visée (5, 5') dans ledit canon creux (11).

Fig-1

Fig-2

6

Fig-3

1"

16
16b 16c 18 20 5'
14' 16a 17 18

Fig-4

1"

16
5'
16b
19 16c

**Office européen des brevets**

# RAPPORT DE RECHERCHE EUROPEENNE

Numéro de la demande

# EP 90 40 3240

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | US-A-4 722 331  (FOX) <br> * En entier * <br><br> − − − | 1-3 | A 61 B 17/16 |
| Y |  | 4,5,7 |  |
| A |  | 15 |  |
| Y | EP-A-0 265 659  (PURNELL) <br> * Colonne 7, ligne 55 - colonne 15, ligne 43; figures 1,2,5,6,9-13 * <br><br> − − − | 4,7 |  |
| A |  | 1,10,14 |  |
| Y | DE-U-8 800 114  (LIXL) <br> * Page 4, lignes 7-13; figures 1c,1d * <br><br> − − − | 5 |  |
| A | FR-A-2 552 655  (S.A.I.D.C.) <br> * Figures 1-4,12-14 * <br><br> − − − | 1,7 |  |
| A | FR-A-2 560 764  (MATCO) <br> * Page 3, lignes 7-9,24-31; figures 1-5 * <br><br> − − − | 5,7 |  |
| A | US-A-4 739 751  (SAPEGA) <br> * Colonne 8, lignes 32-37; figures 4,7A,8A * <br><br> − − − | 7,10 | **DOMAINES TECHNIQUES RECHERCHES (Int. Cl.5)** |
| A | DE-C-3 339 259  (SCHMIEDING) <br> * Colonne 3, lignes 9-57; figure * <br><br> − − − | 9 | A 61 B |
| A | DE-U-8 702 208  (HOWMEDICA) <br> * Page 8, lignes 1-8; figures 1-4 * <br><br> − − − | 11 |  |
| A | FR-A-2 255 040  (RAMBERT) <br><br> − − − |  |  |
| A | FR-A-9 100 78  (FRERET) <br><br> − − − |  |  |
|  | −/− |  |  |

Le présent rapport de recherche a été établi pour toutes les revendications

| Lieu de la recherche | Date d'achèvement de la recherche | Examinateur |
|---|---|---|
| La Haye | 21 décembre 90 | KLEIN C. |

**Office européen
des brevets**

# RAPPORT DE RECHERCHE
# EUROPEENNE

Numéro de la demande

## EP 90 40 3240

| | DOCUMENTS CONSIDERES COMME PERTINENTS | | |
|---|---|---|---|
| Catégorie | Citation du document avec indication, en cas de besoin, des parties pertinentes | Revendication concernée | CLASSEMENT DE LA DEMANDE (Int. Cl.5) |
| A | US-A-2 393 982 (GIESEN)<br>– – – – – | | |
| | | | **DOMAINES TECHNIQUES RECHERCHES (Int. Cl.5)** |

Le présent rapport de recherche a été établi pour toutes les revendications

| Lieu de la recherche | Date d'achèvement de la recherche | Examinateur |
|---|---|---|
| La Haye | 21 décembre 90 | KLEIN C. |